Europäisches Patentamt

European Patent Office

Office européen des brevets

(19)

(11) Numéro de publication: **0 069 035**
A2

# (12) DEMANDE DE BREVET EUROPEEN

(21) Numéro de dépôt: **82420087.7**

(51) Int. Cl.³: **A 61 N 5/04**

(22) Date de dépôt: **30.06.82**

(30) Priorité: **01.07.81 FR 8113075**

(43) Date de publication de la demande: **05.01.83**
**Bulletin 83/1**

(84) Etats contractants désignés: **DE GB NL**

(71) Demandeur: **Etablissement Public dit: CENTRE NATIONAL DE LA RECHERCHE SCIENTIFIQUE (CNRS), 15, Quai Anatole France, F-75007 Paris (FR)**

(72) Inventeur: **Dittmar, André, 8 bis, rue Claude Farrère, F-69003 Lyon (FR)**
Inventeur: **Delhomme, Georges, 27, rue Roger Salengro, F-69500 Bron (FR)**
Inventeur: **Pellissier, Jean-Pierre, 6, rue Salvador Allende, F-69100 Villeurbanne (FR)**
Inventeur: **Schmitt, Michel, 60, rue Waldeck Rousseau, F-69006 Lyon (FR)**

(74) Mandataire: **Ropital-Bonvarlet, Claude et al, Cabinet BEAU DE LOMENIE 99, Grande rue de la Guillotière, F-69007 Lyon (FR)**

(54) **Applicateur de micro-ondes pour la création d'hyperthermies localisées.**

(57) Micro-ondes.

L'applicateur comprend une mase (2) reliée à un guide d'ondes (5) et associée à un échangeur de chaleur (14) en relation avec un circuit (15) de circulation d'un fluide de refroidissement d'une part, et une masse (10) constituée sous la forme d'une enveloppe concentrique à la masse (2) reliée au retour (7) du guide d'ondes et possédant une embasse annulaire (12) associée à un échangeur de chaleur (30) en relation avec un circuit (35) de circulation d'un fluide de refroidissement.

Applications aux traitements des tumeurs cancéreuses.

ACTORUM AG

**CENTRE NATIONAL DE LA RECHERCHE SCIENTIFIQUE (C.N.R.S.)**
**Applicateur de micro-ondes pour la création d'hyperthermies localisées.**

La présente invention concerne les dispositifs utilisés pour créer, à l'intérieur d'une masse déterminée, par l'intermédiaire de micro-ondes, des zones d'hyperthermie localisée.

L'invention concerne, à titre principal, les applicateurs du genre ci-dessus utilisés dans les expérimentations dans le traitement des tumeurs cancéreuses.

Dans le domaine technique ci-dessus, des expérimentations sont menées depuis plusieurs années en vue de tenter le traitement des tumeurs cancéreuses. Ces expérimentations ont mis en évidence l'action spécifique de l'hyperthermie permettant d'observer une régression des tumeurs cancéreuses. Ces expériences ont aussi permis de déterminer que l'hyperthermie accroît la sensibilité des cellules à diverses substances chimiques et permet d'envisager ainsi une potentialisation de la chimiothérapie dans le traitement des tumeurs cancéreuses. En outre, l'hyperthermie accroît apparemment aussi l'effet des radiations ionisantes sur les cellules cancéreuses.

Les micro-ondes peuvent donc, à priori, être retenues comme agent thérapeutique, étant donné qu'elles peuvent pénétrer profondément dans le tissu biologique et atteindre ainsi des tumeurs lointaines.

Les expériences qui ont été menées jusqu'à présent ont fait apparaître, toutefois, un problème fondamental représentant incontestablement un obstacle au développement de telles expérimentations sur le tissu biologique.

En effet, on a constaté que les tissus situés en surface absorbaient, du fait de leur situation de proximité avec l'applicateur, plus d'énergie que les tissus profonds au niveau desquels doit être portée généralement l'hyperthermie en vue du traitement d'une tumeur. Pour obtenir une montée en température suffisante, de l'ordre par exemple de 42 à 44° C en profondeur, on a constaté que les tissus de surface et, notamment la peau, subissaient une montée en température trop élevée conduisant à leur dégradation et imposant de surcroît aux patients une contrainte physique insupportable avec les durées d'irradiation généralement retenues.

Le développement d'une telle méthode et des applicateurs de mise en oeuvre passe donc, nécessairement, par la résolution d'un tel problème, de façon qu'il devienne possible de réaliser une hyperthermie localisée profonde sans pour autant infliger une montée en température élevée et insupportable pour les tissus de surface.

L'objet de l'invention est justement de répondre à ce problème en proposant un nouveau type d'applicateur qui permette de réaliser une régulation de la température des tissus de surface tout en assurant une hyperthermie profonde.

L'objet de l'invention est de proposer un nouvel applicateur qui soit de conception fiable et facilement réalisable à un prix de revient particulièrement intéressant.

Un autre objet de l'invention est de fournir un nouvel applicateur qui ne présente pas d'encombrement nettement plus important que les moyens actuellement connus, tout en permettant d'apporter à volonté une régulation de la température des tissus de surface.

Un objet supplémentaire de l'invention est de mettre en oeuvre des moyens permettant d'atteindre les buts ci-dessus et capables d'assumer une seconde fonction de contrôle de position, de forme au sens général et de dimensions des zones localisées soumises à hyperthermie.

Pour atteindre les buts ci-dessus, l'objet de l'invention est caractérisé par le fait que, dans un applicateur du type comprenant deux masses métalliques de haute conductivité thermique, de polarités différentes, disposées sans contact physique direct l'une à côté de l'autre et dont l'une est associée à un guide d'ondes d'hyperfréquences produit par un générateur,

- l'une des masses est reliée au guide d'ondes et possède, à proximité de sa surface d'application, un échangeur de chaleur en relation avec un circuit de circulation d'un fluide de refroidissement,

- et l'autre masse est constituée sous la forme d'une enveloppe concentrique à la première masse, reliée au retour du guide d'ondes, entourant la première masse sans la toucher et formant au moins une embase annulaire possédant une surface d'application sensiblement située dans le plan de celle de la première masse, ladite surface étant associée à au moins un échangeur de chaleur en relation avec un circuit de circulation d'un fluide de refroidissement.

Diverses autres caractéristiques de l'invention ressortent de la description faite ci-dessous en référence aux dessins annexés qui montrent, à titre d'exemples non limitatifs, des formes de réalisation de l'invention.

La fig. 1 est une coupe-élévation de l'applicateur conforme à l'invention.

La fig. 2 est une vue de dessous prise selon la ligne II-II de la fig. 1.

La fig. 3 est un schéma représentatif des courbes de températures susceptibles d'être obtenues dans un tissu avec un applicateur du type classique.

Les fig. 4 à 7 sont des schémas analogues à celui de la fig. 3, mais mettant en évidence les possibilités de l'objet de l'invention.

La fig. 8 est une vue de dessous analogue à la fig. 2, illustrant une possibilité de variante de réalisation de l'un des éléments constitutifs de l'objet de l'invention.

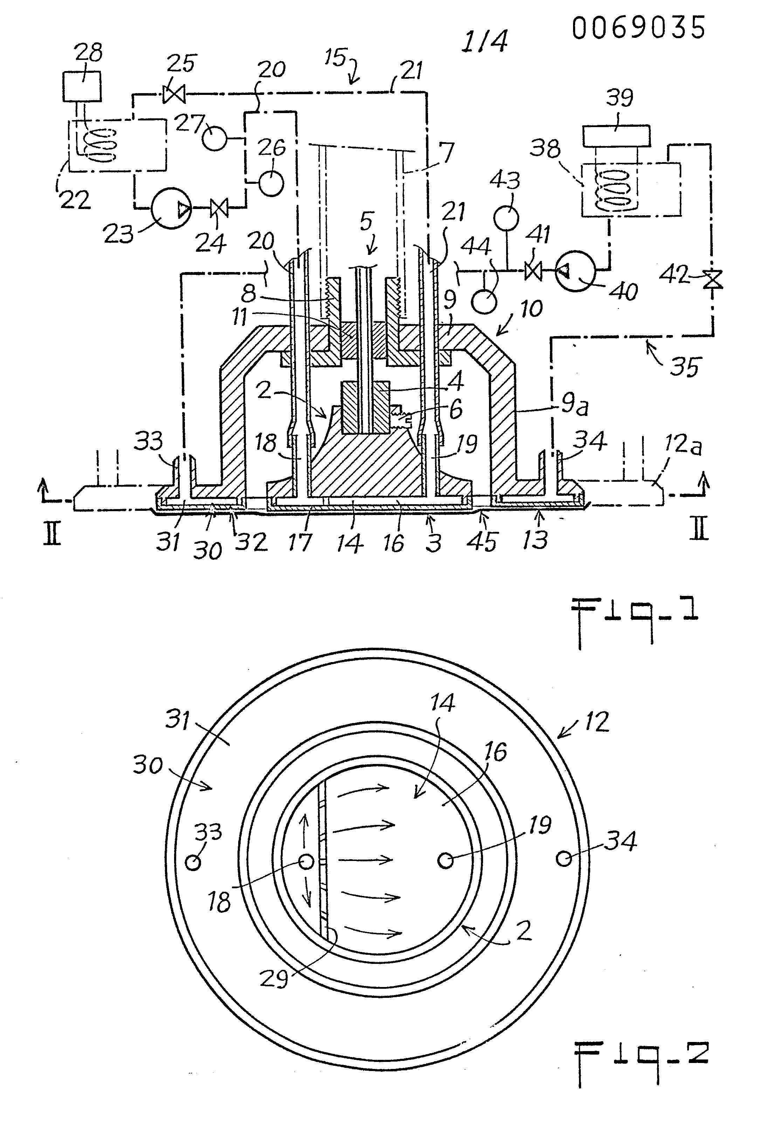

Selon l'exemple de réalisation illustré par les fig. 1 et 2, l'applicateur de micro-ondes, désigné dans son ensemble par la référence 1, comprend, dans le but de créer des hyperthermies localisées, une première masse 2 de haute conductivité thermique, de préférence réalisée en alliage de métaux légers, tels qu'en duraluminium. Cette masse 2 possède, à l'opposé d'une surface 3 d'application sur le tissu à traiter, une bague 4 en matière conductrice assurant le raccordement avec un guide d'ondes 5, par exemple constitué par un conducteur coaxial. La liaison entre la bague 4 et la masse 2 peut être rendue réglable par l'intermédiaire d'une vis de serrage 6. Le conducteur coaxial 5 possède une gaine 7, dite de retour, qui est fixée et centrée sur une douille 8 en matière conductrice, traversant le dessus 9 d'une seconde masse 10 en matière à haute conductivité thermique, par exemple identique à celle constitutive de la masse 2. La liaison entre la gaine de retour 7, la douille 8 et la masse 10 est mise à profit pour assurer le centrage du conducteur coaxial 5 par l'intermédiaire d'un manchon de centrage 11 en matière isolante.

La seconde masse 10 est réalisée sous la forme d'une cloche comportant, à partir du dessus 9, une enveloppe 9a, par exemple circulaire, qui s'étend coaxialement à la masse 2, sans posséder de contact physique avec cette dernière. L'enveloppe 9a forme, au niveau de son bord inférieur, une embase annulaire 12 possédant une surface d'application 13 s'étendant sensiblement dans le même plan que la surface 3. La masse 9 peut être réalisée en une ou plusieurs parties.

La masse 2 est associée à un échangeur de chaleur 14 qui est en relation avec un circuit 15 de circulation d'un fluide de refroidissement. L'échangeur de chaleur 14 est, de préférence, formé par un évidement 16 ménagé dans la masse 2 à partir de la surface d'application 3, sur une faible profondeur constante. L'évidement 16 présente en plan, tel que cela apparait par la fig. 2, la forme d'une cavité cylindrique. L'évidement 16 est fermé par une plaque ou couvercle 17 amovible, réalisé en une matière à haute conductivité thermique et qui peut être emboîté, vissé, soudé ou rapporté de toute autre façon convenable de manière à établir une étanchéité certaine de la fermeture de l'évidement 16 constituant l'échangeur de chaleur 14. La surface extérieure du couvercle 17 représente alors la face d'application 3.

La masse 2 comporte également au moins deux tubulures de raccordement 18 et 19 qui peuvent être constituées par des segments tubulaires, par exemple en matière plastique, rapportés dans la masse 2 pour déboucher à l'intérieur de l'évidement 16. Les tubulures 18 et 19 assurent le raccordement avec deux canalisations 20 et 21 faisant partie du circuit 15. Les canalisations 20-21 sont maintenues au niveau de la masse 10 en traversant le fond de l'enveloppe 9a. Les canalisations 20 et 21 représentent, respectivement, un circuit de retour et un circuit d'amenée pour un fluide de refroidissement, notamment de l'eau, prélevé à partir d'un réservoir-tampon 22 par une pompe 23. Le circuit 15 comporte, outre des vannes 24 et 25 d'isolement, un débimètre 26 et un thermomètre 27. Le réservoir-tampon 22 peut être associé à un circuit refroidisseur, désigné dans son ensemble par la référence 28.

La fig. 2 montre que, de préférence, les tubulures 18 et 19 débouchent de façon diamétralement opposées dans l'échangeur 14. Eventuellement, un perturbateur interne 29 peut être prévu pour favoriser un écoulement réparti et une meilleure circulation du fluide de refroidissement à l'intérieur de l'échangeur 14, et accroître ainsi les capacités d'échange de ce dernier en évitant toute zone de moindre circulation ou toute zone morte.

L'embase annulaire 12 de la seconde masse 10 est également associée à un échangeur de chaleur 30, de préférence, formé par un évidement 31 ménagé à partir de la face 13. L'évidement 31 est délimité sur une profondeur constante, à la manière d'une chambre ou cavité annulaire. L'évidement 31 est fermé par un couvercle 32 en matière à conductibilité élevée, qui peut être emboîté de façon amovible ou rendu solidaire de l'embase 12 par vissage, collage, soudage ou tout autre moyen approprié, pour établir une fermeture étanche. La face extérieure du couvercle annulaire 32 représente alors la surface d'application 13.

L'échangeur de chaleur 30 est placé en relation, par des tubulures 33 et 34 formées par ou rapportées sur l'embase annulaire 12, avec un circuit de circulation 35 d'un fluide de refroidissement, tel que de l'eau. Le circuit 35 comporte deux canalisations 36 et 37 représentant des circuits, respectivement, de retour et d'amenée, par rapport à un réservoir-tampon 38, éventuellement associé à un dispositif refroidisseur. Le circuit 35 comporte également une pompe de circulation 40, deux vannes d'isolement 41 et 42, ainsi qu'un débimètre 43 et un thermomètre 44.

Selon une autre disposition de l'invention, le maintien concentrique des masses 2 et 10 est assuré par l'intermédiaire d'une feuille ou d'un film 45 en matière isolante, rapporté, de façon adhésive, pour couvrir sans interruption les faces extérieures des couvercles 17 et 32 situées sensiblement sur le même plan.

Il peut être prévu, également, de disposer à l'intérieur de l'échangeur 30 un ou des perturbateurs de circulation comme pour l'échangeur 14.

Le fonctionnement de l'applicateur peut être décrit de façon simplifiée pour ce qui concerne le cheminement des micro-ondes. En effet, le conducteur coaxial 5 assure le cheminement des micro-ondes fournies par le générateur, par exemple un magnétron, jusqu'au niveau de la masse 2 à l'intérieur de laquelle elles se répartissent pour suivre, par la périphérie, le couvercle 17 permettant le bouclage et le retour par le couvercle annulaire 32, l'embase 12 et la masse 10, elle-même reliée à la gaine de retour 7.

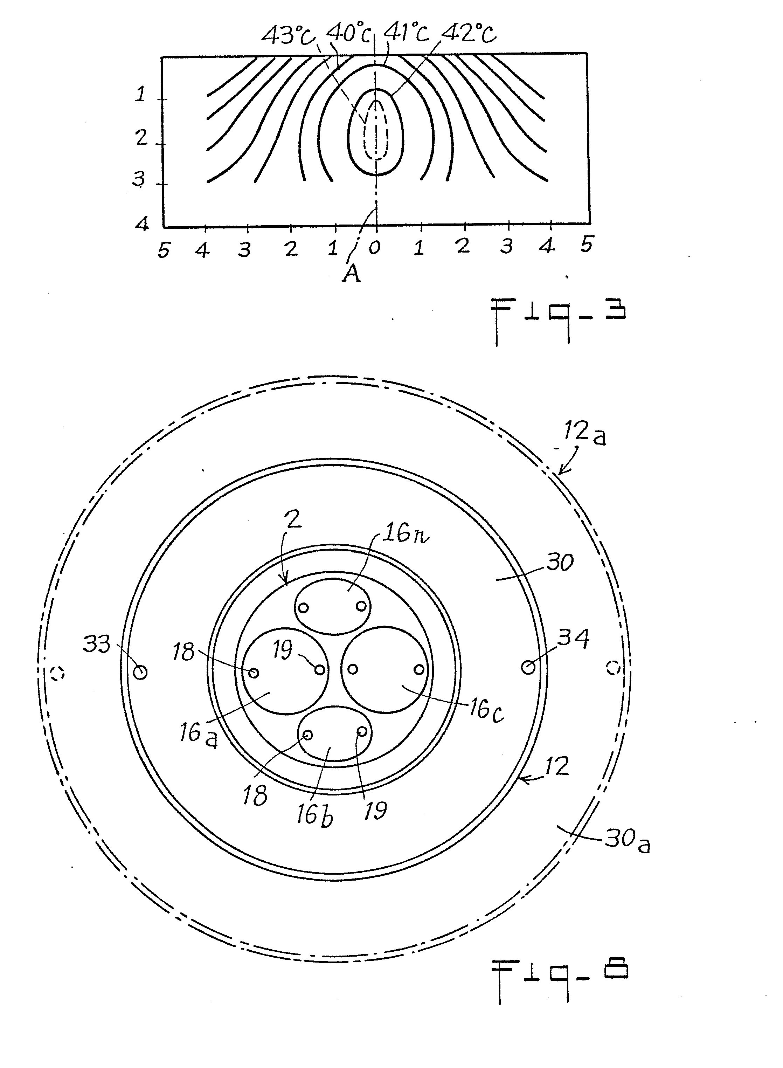

L'application d'un tel applicateur sur un tissu, par exemple biologique, permet alors de créer une montée en température ou hyperthermie localisée, du type de celle illustrée schématiquement par la fig. 3, dans la mesure où les conditions suivantes sont, par exemple, réunies :

- température ambiante .......................... 20° C
- puissance appliquée au générateur d'ondes
  d'hyperfréquences ............................. 15 watts
- gamme de fréquence retenue ................... 2 450 MHz

Selon ce schéma, on constate qu'une hyperthermie induite localisée, de l'ordre de 43° C, est créée dans une zone comprise entre 1 et 3 cm de profondeur par rapport à la surface d'application avec un rayonnement radial de l'ordre de 0,5 cm par rapport au centre de l'applicateur désigné par l'axe A. On constate aussi, par les différents isothermes, que la seule application, comme dit ci-dessus, conduit à soumettre les tissus de surface à une élévation de température jusqu'à environ 40° C.

Un tel applicateur ne peut donc valablement être mis en oeuvre compte tenu du risque de contrainte physique et de détérioration, notamment de brûlures, infligées aux couches superficielles du tissu traité.

La fig. 4 montre la mise en oeuvre du même dispositif décrit ci-dessus, en faisant intervenir cette fois les moyens prévus selon l'invention pour réaliser un refroidissement localisé superficiel de la zone à traiter. Dans un tel exemple, les paramètres suivants ont été retenus :

- puissance micro-ondes ......................    10 watts
- fréquence retenue ..........................    2 450 MHz
- température des fluides de refroidissement ....    20° C
- débit circuit 15 ...........................    10 ml/mn
- débit circuit 35 ...........................    10 ml/mn

L'examen de la fig. 4 permet de constater que ces conditions aboutissent à une hyperthermie induite de forme, dimensions, localisation et profondeur, notablement différentes de celles selon la fig. 3. On constate, en effet, une température sensiblement égale à 30° C située à une profondeur comprise entre 2,5 et 4 cm dans une zone comprise entre 0,5 et 2 cm de rayonnement radial à partir de l'axe A. Cette figure permet également de constater que la température de surface est sensiblement de l'ordre de 13° C et connaît, par conséquent, une montée en température notablement inférieure à celle de l'hyperthermie localisée en profondeur.

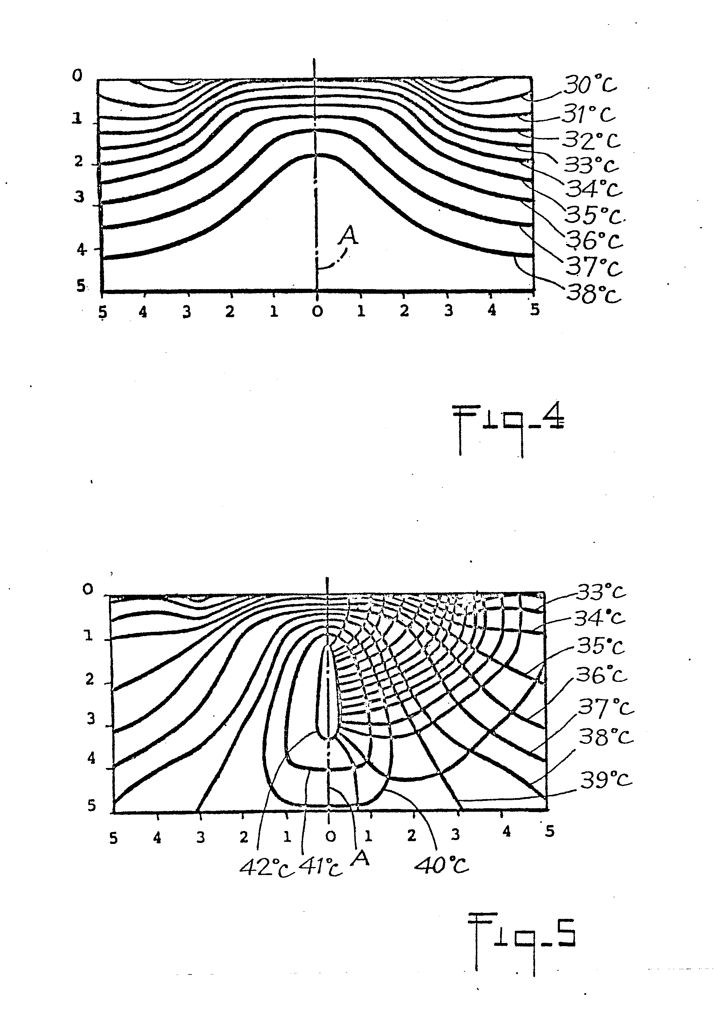

La fig. 5 montre un exemple d'hyperthermie correspondant à une mise en oeuvre de l'applicateur en vue du traitement d'une cellule cancéreuse.

Dans cet exemple, les conditions suivantes ont été réunies :

- puissance micro-ondes ......................    20 watts
- fréquence retenue ..........................    2 450 MHz
- température des liquides de refroidissement .....    20° C
- débit circuit 15 ...........................    15 ml/mn

- débit circuit 35 ............................... 20 ml/mn

L'examen de la fig. 5 permet de constater l'existence d'une hyperthermie localisée de température de l'ordre de 42° C en localisation du type fermé, située entre 1 et 5 cm de profondeur par rapport aux couches superficielles et rayonnant sur environ 1,5 cm à partir de l'axe A. Cette figure permet également de constater que, dans les conditions ci-dessus, la couche superficielle du tissu traité fait apparaître une température de l'ordre de 33° C.

La comparaison des fig. 4 et 5 permet ainsi de constater que la circulation du fluide de refroidissement dans les échangeurs 14 et 30 a pour effet de prélever les calories résultant de l'absorption des couches superficielles et, par conséquent, de maintenir ces dernières à une température convenable optimale et acceptable, tout en créant en profondeur l'hyperthermie recherchée. Selon les conditions d'utilisation de l'applicateur, c'est-à-dire la puissance et la fréquence retenues, il devient donc possible, en modifiant les paramètres de circulation des circuits 15 et 35, de faire régner au niveau des couches superficielles des températures acceptables indépendantes de l'hyperthermie profonde.

La comparaison des fig. 4 et 5 permet aussi de constater que l'embase annulaire 12 assume une seconde fonction en plus de celle de refroidissement. En effet, en modifiant les conditions de circulation pour le circuit 35, il devient possible de modeler la forme de l'hyperthermie induite, par exemple en accroissant la fonction de refroidissement pour rabattre les isothermes de basse valeur sur la partie axiale de l'hyperthermie induite. De la sorte, l'embase annulaire 12 assume une fonction de focalisateur thermique, limitant les propagations latérales des hyperthermies profondes et permettant ainsi la création d'une zone d'hyperthermie, par exemple de forme allongée dans le sens de la hauteur selon l'exemple de la fig. 5. Il devient ainsi possible, en modifiant de façon correspondante les paramètres, de choisir la conformation en zone fermée ou ouverte de l'hyperthermie induite pour la faire correspondre en volume le mieux possible à celui de la cellule devant être traitée.

La fig. 5 montre aussi les lignes de flux thermique résultant du prélèvement de calories par les échangeurs de chaleur.

La fig. 6 montre un exemple de conformation différente d'une hyperthermie induite faisant intervenir les conditions d'application suivantes :

- puissance micro-ondes ........................ 20 watts
- fréquence ................................... 2 450 MHz
- température des fluides de refroidissement... 20° C
- débit circuit 15 ........................... 30 ml/mn
- débit circuit 35 ........................... 20 ml/mn

Avec de telles conditions, une température de surface de l'ordre de 32° C est atteinte, alors qu'une hyperthermie localisée de l'ordre de 39° C est établie sur une profondeur de 1 à 4 cm et sur environ 1 cm de rayonnement à partir de l'axe A.

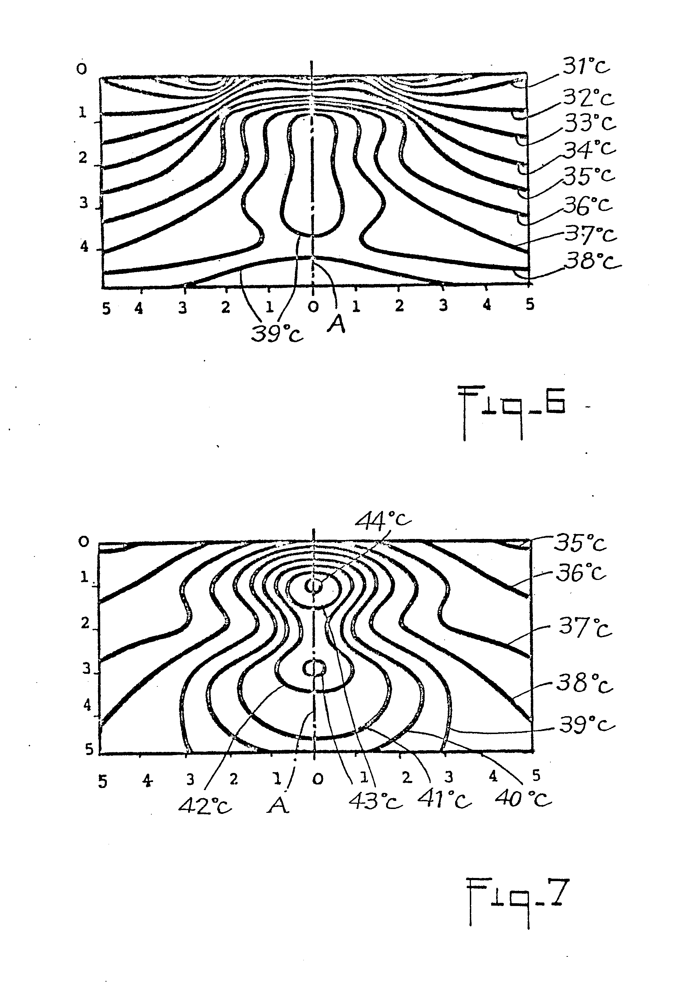

La fig. 7 montre un résultat différent obtenu avec les conditions suivantes :

- puissance micro-ondes ........................ 75 watts
- fréquence ................................... 2 450 MHz
- température du fluide de refroidissement ..... 20° C
- débit circuit 15 ........................... 25 ml/mn
- débit circuit 35 ........................... 50 ml/mn

Avec des conditions telles que celles ci-dessus, une température de surface de 38° C a été obtenue avec création d'une hyperthermie en zone fermée mais à double foyer, possédant une température de l'ordre de 44° C à environ 1 cm de profondeur sur environ 1 cm de rayonnement à partir de l'axe A et une température de 43° C établie à environ 3 cm de profondeur avec un rayonnement de l'ordre de 1 cm à partir de l'axe A.

Les moyens selon l'invention permettent, par conséquent, de réduire sensiblement la température de surface du tissu d'application, tout en établissant une hyperthermie de condition recherchée dans les couches profondes et, supplémentairement, de conférer à cette hyperthermie une zone globale ou volume virtuel d'influence, de conformation ouverte ou fermée, à simple ou double foyer, pouvant, par conséquent, être adaptée sensiblement à la localisation et la conformation d'une tumeur identifiée devant être traitée.

Comme dit précédemment, la disposition coaxiale des masses 2 et 10, qui peuvent être qualifiées, respectivement, de de conducteur et refroidisseur central et de conducteur et refroidisseur périphérique, est établie par l'intermédiaire de la feuille ou du film 45. De préférence, ce film est choisi en matière plastique de faible épaisseur, de l'ordre de 0,1 mm. Il en résulte une conductance thermique élevée dans le sens axial, étant donné la faible épaisseur et la grande surface relative. Le film 45 ne freine donc pas les échanges thermiques entre l'applicateur et les tissus à traiter. Par contre, la conductance thermique du film dans le sens radial est très faible, de sorte que les échanges thermiques, entre le refroidisseur central et le focalisateur thermique, sont réduits à la valeur minimale. Il y a lieu de noter que le film 45 assume la fonction supplémentaire de protection électrique du tissu, étant donné qu'il recouvre toutes les parties métalliques de haute conductivité susceptibles d'être amenées en contact avec le tissu biologique à traiter.

Il importe de souligner que la disposition constructive de l'objet de l'invention permet de réaliser un bouclage complet au niveau de chaque échangeur de chaleur 14 ou 30, par l'intermédiaire de la masse et de la plaque 17 ou 32 en matière conductrice. De la sorte, les micro-ondes trouvent un circuit conducteur naturel et peuvent se refermer autour des cavités 16 et 31 dans lesquelles circulent les lames de fluide de refroidissement, sans qu'il en résulte un échauffement de ce dernier.

La partie centrale est représentée sous la forme d'une masse compacte, mais il doit être considéré que, dans le but de réduire l'inertie thermique, il est possible de prévoir une réalisation faisant, par exemple, intervenir un évidement central ne laissant subsister qu'un circuit de cheminement pour les micro-ondes.

Pour un applicateur mettant en oeuvre les paramètres, tels que fournis ci-dessus, il peut être retenu de donner à l'évidement 16 ou 31 une profondeur de 0,7 mm permettant d'obtenir des vitesses linéaires moyennes de circulation supérieures à 20 mm/s pour un débit de refroidissement de 0,5 $cm^3$/s. Une telle vitesse linéaire moyenne est favorable aux échanges thermiques et donne une réponse rapide. Dans ce but, également, il peut être prévu de conférer aux plaques 17 et 32 une épaisseur de l'ordre de 0,3 mm.

La fig. 8 montre que l'échangeur 14 peut comporter plusieurs évidements, 16a-16n, possédant, soit un couvercle commun, soit des couvercles de fermeture individuels en étant alimentés chacun par des tubulures propres à partir du circuit de circulation 15.

Les fig. 1 et 8 montrent, en traits mixtes, qu'il peut être prévu de réaliser la masse 10 pour lui faire comporter une embase annulaire 12a, susceptible de supporter deux échangeurs 30 et 30a côte à côte, indépendants l'un de l'autre.

L'invention n'est pas limitée aux exemples décrits et représentés, car diverses modifications peuvent y être apportées sans sortir de son cadre. En particulier, il peut être prévu de faire circuler les fluides de refroidissement des deux masses selon des sens opposés.

12 0069035

REVENDICATIONS :

1 - Applicateur de micro-ondes pour la création d'hyperthermies localisées du type comprenant deux masses métalliques de haute conductivité thermique, de polarités différentes, disposées sans contact physique direct l'une à côté de l'autre et dont l'une est associée à un guide d'ondes d'hyperfréquences produites par un générateur, caractérisé en ce que :

- l'une des masses (2) est reliée au guide d'ondes (5) et possède, à proximité de sa surface d'application (3), un échangeur de chaleur (14) en relation avec un circuit (15) de circulation d'un fluide de refroidissement,

- et l'autre masse (10) est constituée sous la forme d'une enveloppe (9a) concentrique à la première masse, reliée au retour (7) du guide d'ondes, entourant la première masse sans la toucher et formant au moins une embase annulaire (12) possédant une surface d'application (13) sensiblement située dans le plan de celle de la première masse, ladite surface étant associée à au moins un échangeur de chaleur (30) en relation avec un circuit (35) de circulation d'un fluide de refroidissement.

2 - Applicateur selon la revendication 1, caractérisé en ce que les échangeurs de chaleur (14-30) des deux masses sont constitués chacun par au moins une cavité ou chambre (16 ou 31), ménagée à l'intérieur de la masse et reliée, par au moins deux embouts de raccordement (18-19 ou 33-34), à une circulation d'un fluide de refroidissement.

3 - Applicateur selon la revendication 1 ou 2, caractérisé en ce que les échangeurs (14-30) des deux masses sont reliés à des circuits de circulation (15-35) différents possédant chacun des moyens propres de régulation.

4 - Applicateur selon l'une des revendications 1 à 3, caractérisé en ce que l'un au moins des échangeurs est formé par au moins une chambre ou cavité contenant au moins un perturbateur de circulation (29).

5 - Applicateur selon la revendication 2, caractérisé en ce que l'un au moins des échangeurs est constitué par au moins une chambre ou cavité formée par un évidement (16) ménagé dans la masse correspondante à partir de la surface d'application et fermé par un couvercle (17) en forme de plaque conductrice.

6 - Applicateur selon la revendication 1, caractérisé en ce que la masse (10) en forme d'enveloppe concentrique possède une conformation du type cloche assumant une fonction de support du guide d'ondes (5) et des canalisations (20-21) de circulation du fluide de l'échangeur (14) de la masse centrale (2).

7 - Applicateur selon la revendication 1, caractérisé en ce que les surfaces d'application (3-13) des deux masses sont réunies par une feuille ou un film (45) de matière isolante.

8 - Applicateur selon la revendication 1, caractérisé en ce que la masse centrale est évidée dans sa partie centrale.

1/4    0069035

Fig-1

Fig-2

Fig-3

Fig-8

Fig-4

Fig-5

4/4

0069035

Fig-6

Fig-7